# EUROPÄISCHE PATENTSCHRIFT

(11) **EP 1 565 176 B1**
(45) Veröffentlichungstag und Bekanntmachung des Hinweises auf die Patenterteilung: **24.05.2006**
(21) Anmeldenummer: 03778338.8
(22) Anmeldetag: 13.10.2003
(51) Int. Cl.: A61K 31/194, A61K 31/341, A61K 31/351, A61K 31/05, A61K 31/198, A61P 35/00

(54) **MITTEL MIT ZERSTÖRENDER WIRKUNG AUF MALIGNE TUMORE SOWIE VERFAHREN ZU SEINER HERSTELLUNG**
AGENT HAVING A DESTRUCTIVE EFFECT ON MALIGNANT TUMORS AND METHOD FOR THE PRODUCTION THEREOF
COMPOSÉ A ACTION DESTRUCTRICE SUR DES TUMEURS MALIGNES ET SON PROCÉDÉ DE PRODUCTION

(30) Priorität: 27.11.2002 AT 17782002
(43) Veröffentlichungstag der Anmeldung: 24.08.2005
(73) Patentinhaber: C.Y.L. Pharmazeutika GmbH, 8501 Lieboch (AT); Groke, Karl, 8063 Eggersdorf (AT)
(72) Erfinder: GROKE, Karl, A-8063 Eggersdorf (AT); HERWIG, Ralf, A-6363 Westendorf (AT); Ferdinand, Peter., A-8043 Graz (AT)
(74) Vertreter: Müllner, Erwin
(86) Internationale Anmeldenummer: PCT/EP2003/050712
(87) Internationale Veröffentlichungsnummer: WO 2004/047832

(56) Entgegenhaltungen:
- WO-A-00/67762
- DE-A- 3 542 309
- US-A- 5 006 551
- KARNBROCK W ET AL: "A new efficient synthesis of acetyltelluro- and acetylselenomethionine and their use in the biosynthesis of heavy-atom protein analogs" JOURNAL OF THE AMERICAN CHEMICAL SOCIETY 1996 UNITED STATES, Bd. 118, Nr. 4, 1996, Seiten 913-914, XP002270592 ISSN: 0002-7863 in der Anmeldung erwähnt

## Beschreibung

### TECHNISCHES GEBIET

Die vorliegende Erfindung betrifft ein Mittel mit zerstörender Wirkung auf maligne Tumore, das als Wirkstoffe alpha-Ketoglutarsäure oder deren pharmazeutisch verträgliche Salze und mindestens eine in enzymunabhängiger Reaktion zur Azomethinbildung befähigte Verbindung aus der Gruppe 5-Hydroxymethylfurfural, Dehydroascorbinsäure, Maltol und Vanillin umfasst, wobei vorzugsweise das Masseverhältnis der Ketoglutarsäure zu der mindestens einen zur Azomethinbildung befähigten Verbindung größer als 1:1, insbesondere 2:1 bis 12:1, ist.

### STAND DER TECHNIK

Solch ein Mittel ist in der EP 326826 B1 beschrieben.

Es ist bekannt, dass bei einer Reihe von Patienten mit malignen Tumoren eine hochsignifikante Vermehrung von alpha-Ketoglutarsäure im Blut feststellbar ist. Dieser erhöhte Blutspiegel beruht auf einer Störung des Zitronensäurezyklus in den Zellen des Tumors, die zu einer Anreicherung von alpha-Ketoglutarsäure im Tumor führt. Da die alpha-Ketoglutarsäure durch ihren stark sauren pH-Wert für den Tumor schädlich ist, wird diese Säure in vermehrtem Maße vom Tumor ausgeschieden, wobei das Vorhandensein von Ammoniak oder Aminen dieser Ausschleusung dient.

Die Wirkung des in der EP 326826 B1 beschriebenen Mittels beruht darauf, dass alpha-Ketoglutarsäure in höherer Konzentration Tumorzellen zerstört. Es genügt aber nicht, alpha-Ketoglutarsäure dem Tumor zuzuführen, man muss auch die Ausschleusung der alpha-Ketoglutarsäure aus dem Tumor möglichst unterbinden. Dieses Mittel enthält daher als Wirkstoff nicht nur alpha-Ketoglutarsäure, durch deren Verabreichung der hohe Blutspiegel noch zusätzlich erhöht wird, sondern auch einen weiteren Wirkstoff, der in enzymunabhängiger Reaktion Ammoniak und Amine unter Azomethinbildung zu binden vermag, die dann für die Ausschleusung der alpha-Ketoglutarsäure fehlen, sodass die Ausschleusung der alpha-Ketoglutarsäure aus dem Tumor dadurch gehemmt wird. Als solche Substanzen werden in der EP 326826 B1 5-Hydroxymethylfurfural, Dehydroascorbinsäure, Maltol und Vanillin genannt, wobei 5-Hydroxymethylfurfural besonders bevorzugt ist. Die Anreicherung der alpha-Ketoglutarsäure im Tumor hat zur Folge, dass es zu einer bis zur völligen Eliminierung des Tumors führenden Schädigung desselben kommt. Voraussetzung ist hierbei, dass die maligne Erkankung mit einer Erhöhung des alpha-Ketoglutarsäurespiegels im Blut des Patienten einhergeht. Die Wirkung des Präparates gemäß AT 393.221 B1 wurde anhand von klinischen Studien bestätigt.

Es ist auch bekannt, dass Selenverbindungen bei Patienten, die an bestimmten malignen Erkrankungen leiden, eine begünstigende Wirkung besitzen. So wird in der Publikation von A. Pakdaman in Biol.-Trace-Elem-Res. 1998 April-May; 62 (1-2) 1-6 beschrieben, dass die Verabreichung einer Selenverbindung, nämlich Na-Selenit, an Patienten, die an einem malignen Gehirntumor leiden, zu einer Verbesserung des Allgemeinbefindens der Patienten führt, dass sich unter anderem bei allen behandelten Patienten auch eine Besserung der Zahl an Erythrocyten, eine Besserung der Hämoglobinwerte und der Zahl der Thrombocythen einstellt. 76% dieser Patienten verspürten außerdem ein Abnehmen von unangenehmen Nebenwirkungen. Als besonders vorteilhaft wird die Anwendung von Selenit bei jenen Patienten mit Gehirntumor beschrieben, die die Oxygen-Therapie erhielten. Ob bei dieser Medikation auch die Krebserkrankung selbst gebessert wird, ist nicht erwähnt. An sich ist zu Selenverbindungen gemäß der Literatur zu sagen, dass sie in erster Linie als Mittel zur Verhütung von Krebs angesehen werden. Sie werden nicht als Therapeutikum zur Behandlung von Krebs betrachtet, sondern als Mittel zur Verhütung von Krebs. Sie werden dabei zumeist als Nahrungsergänzung angesehen und mit Gemüsesorten kombiniert. So wurden in der Literatur z.B. Kombinationen mit Knoblauch, Zwiebel oder Broccoli genannt.

Mit der Krebsvorbeugung befassen sich auch Jian C., Jiang W., Ip C1., Ganther H. und Lu J. in ihrer Arbeit "Seleniuminduced inhibition of Angiogenesis in Mammary Cancer at chemopreventive levels of intake". Molecular Carcinogenesis 26,213-225, (1999).

In dieser Arbeit wurde untersucht, ob die Krebsvorbeugung darauf zurückzuführen sei, dass die krebsvorbeugende Wirkung von Selen zumindest zum Teil durch Hemmung von mit einem Krebsgeschehen assoziierter Angiogenese verbunden sein könnte. Für diese Untersuchungen wurden Ratten herangezogen, die durch Verabreichung von I-Methyl-I-nitrosoharnstoff ein Mammacarcinom entwickelt hatten. An diesen Ratten wurde die Dichte von Mikro-Gefäßen untersucht, wenn den Ratten Selen in Form von Knoblauch, der mit Selen angereichert wurde, als Natrium-selenit oder als Se-methylselenocystein verabreicht wurde. Dabei konnte festgestellt werden, dass die Dichte der intratumoralen Mikro-Gefäße im Mammacarcinom signifikant reduziert worden war. Zu der Substanz Se-methyl-selenocystein wurde keine Angabe über eine Herstellungsmöglichkeit genannt, sondern nur festgestellt, dass diese Substanz ein Bestandteil des in Knoblauch angereicherten Selens sei. Man sah an diesen Versuchen, die in vitro und in vivo durchgeführt wurden, eine Möglichkeit, dass die antiangiogene Aktivität der Mechanismus der krebsvorbeugenden Wirkung sein könnte.

In ihren Bemühungen, das Präparat gemäß EP 326826 B1 zu optimieren, stießen die Erfinder der vorliegenden Anmeldung auf die Veröffentlichung von Jiang C. et al., die oben zitiert ist, und stellten insofern eine gewisse Parallelität mit dem Mittel gemäß der oben zitierten Patentschrift fest, als die antiangiogene Wirkung von Selenverbindungen, die im Falle von Krebserkrankungen die Dichte der Mikrogefäße des Tumors reduziert, zu einer Einschränkung in der Versorgung des Tumors führt.

### OFFENBARUNG DER ERFINDUNG

Da die Erfinder der vorliegenden Anmeldung vermuteten, dass die antiangiogene Aktivität von Selenverbindungen ebenfalls einen Weg zur Zerstörung des Tumors darstellen könnte, wurden Untersuchungen durchgeführt, die zeigen sollten, ob die Wirkung der Selenverbindungen auch bei anderen Krebsformen zu einer Störung der Angiogenese im Tumor führen würde oder nicht. Ferner musste untersucht werden, in welcher Form Selen dem Präparat gemäß der EP 326826 B1 zugesetzt werden könnte.

So wurde zunächst versucht, Selen in Form von Selenit einzusetzen. Diese Substanz erwies sich jedoch als nicht brauchbar, weil sofort eine Reaktion der alpha-Ketoglutarsäure mit dem Selenit einsetzte, die zur Folge hatte, dass amorphes Selen ausfiel. Man hoffte jedoch, diese Redoxreaktion unterbinden zu können, wenn Selen in organischer Bindung eingesetzt wird. Als eine solche Verbindung wurde schließlich Selenomethionin herangezogen. Ein Versuch ergab, dass diese Selenverbindung mit der alpha-Ketoglutarsäure nicht reagierte. Es zeigte sich jedoch, dass auf Grund der freien Aminogruppe des Selenomethionins diese mit der Ketoglutarsäure die Maillardreaktion einging, die bei höheren Temperaturen, wie sie bei der Hitzesterilisation benötigt wird, besonders begünstigt werden würde.

Überraschenderweise wurde nun gefunden, dass die Substanz N-Acetyl-seleno-L-methionin alle erhobenen Forderungen hinsichtlich einer Verträglichkeit mit der alpha-Ketoglutarsäure erfüllte. Es zeigte sich jedoch, dass bei Einsatz dieser Verbindung als Ergänzung zur Rezeptur gemäß EP 326826 B1 die Gefahr eines Einbaues dieser Substanz in Körperproteine bestand, wodurch die erhoffte Wirkung des N-Acetyl-seleno-L-methionins geschmälert bzw. vollständig verloren gehen würde. Überraschenderweise zeigte sich jedoch, dass der Zusatz N-Acetyl-L-methionin in einer Menge, die etwa das Hundertfache der Menge an N-Acetyl-seleno-L-methionins beträgt, den Einbau von N-Acetyl-seleno-L-methionin in Proteine unterbindet. Bei Versuchen auf Basis der durch die Vorarbeiten sich ergebenden Rezeptur zeigte sich unerwarteterweise, dass das N-Acetyl-seleno-L-methionin zusammen mit den Wirkstoffen der Zusammensetzung gemäß EP 326826 B1 eine Wirkungssteigerung bewirkt, die über eine additive Wirkung hinausgeht und überraschenderweise als Synergismus einzustufen ist.

Gegenstand der vorliegenden Erfindung ist demnach ein Mittel der eingangs genannten Art, das als weitere Wirkstoffe N-Acetyl-seleno-L-methionin und N-Acetyl-L-methionin enthält, wobei Letzteres im Überschuss gegenüber Ersterem vorliegt.

Das erfindungsgemäße Mittel enthält somit (zumindest) vier Wirkstoffe, nämlich alpha-Ketoglutarsäure, eine oder mehrere Verbindungen, die zur Azomethinbildung mit Ammoniak oder Aminen, die im Tumor vorhanden sind und unter anderem der Ausschleusung der alpha-Ketoglutarsäure aus dem Tumor dienen, befähigt sind, N-Acetyl-seleno-L-methionin und N-Acetyl-L-methionin. Dieses Mittel ist in der Lage, das Ausschleusen von alpha-Ketoglutarsäure aus dem Krebsgewebe zu verhindern und dadurch einen hohen Spiegel an dieser Säure im Tumorgewebe zu erzeugen, der letzlich zur Vernichtung des Tumors führt. Als zur Azomethinbildung befähigte Verbindung ist besonders 5-Hydromethylfurfural hervorzuheben.

Es ist günstig, wenn das Masseverhältnis von alpha-Ketoglutarsäure zu N-Acetyl-seleno-L-methionin 100:1 bis 20000:1, vorzugsweise 500:1 bis 10000:1, beträgt und das Masseverhältnis von N-Acetyl-L-methionin zu N-Acetyl-seleno-L-methionin 20:1 bis 300:1, vorzugsweise 50:1 bis 100:1, beträgt.

Das N-Acetyl-seleno-L-methionin ist als Substanz in der Literatur beschrieben und kann z.B. nach W. Karnbrock et al. J.Am.Chem.Soc. (1996), 118 (4), 913-14 mit guter Ausbeute hergestellt werden. Eine Verwendung dieser Substanz als Wirkstoff in der Krebstherapie ist bisher nicht beschrieben worden. Auch eine andere Indikation ist nicht gefunden worden.

Das erfindungsgemäße Mittel entfaltet vor allem dann eine besonders gute Wirkung, wenn das Masseverhältnis der alpha-Ketoglutarsäure zur zur Azomethinbildung befähigten Substanz 2:1 bis 12:1 beträgt.

Es hat sich ferner als günstig erwiesen, dem erfindungsgemäßen Mittel ein Monosaccharid, insbesondere Glucose, Fructose oder ein Gemisch derselben, zuzusetzen, da die sauren Metabolite derselben die Wirkung der alpha-Ketoglutarsäure auf den malignen Tumor unterstützen und überdies auch eine stabilisierende Wirkung auf das Mittel ausgeübt wird. Bei Mitteln, die nicht der intravenösen Verabreichung dienen, können anstelle von Monosacchariden auch Disaccharide zugesetzt werden. Auch diese Disaccharide wirken stabilisierend und sind bei oral zu verabreichenden Mitteln auch geschmacksverbessernd.

Unter den zur Azomethinbildung befähigten Verbindungen ist das 5-Hydroxymethylfurfural am besten geeignet.

Wenn das Mittel in wässriger Lösung vorliegt, soll das N-Acetyl-seleno-L-methionin vorzugsweise in einer Menge von 1,4-2,3 mg/l und das N-Acetyl-L-methionin in einer Menge von 70-230 mg/l vorliegen. Es ist nicht zielführend, bei einer hohen Menge von alpha-Ketoglutarsäure, z.B. von 16 g/l, die Menge an N-Acetyl-seleno-L-methionin über die 2,3 mg/l hinaus zu erhöhen. Natürlich wird diese Selenverbindung jedoch innerhalb des Bereiches von 1,4-2,3 mg/l der alpha-Ketoglutarsäuremenge im Präparat angepasst. Sie beträgt üblicherweise etwa 0,1 Masse-%. Die einzusetzende Menge an N-Acetyl-L-methionin wird innerhalb der angegebenen Grenzen nach der vorliegenden Menge der Selenoverbindung gewählt.

Die Anwesenheit von hohen Dosen an alpha-Ketoglutarsäure, die stark sauer reagiert, birgt die Gefahr in sich, dass die drei weiteren Wirkstoffe des erfindungsgemäßen Präparates ganz oder teilweise zersetzt werden und dadurch der Wirkungsgrad und die Stabilität beeinträchtigt werden. Es ist daher zu empfehlen, den pH-Wert des erfindungsgemäßen Mittels auf einen physiologischen Bereich einzustellen. Besonders vorteilhaft ist ein pH-Bereich von 4-6. Dabei ist darauf zu achten, dass eine Einstellung des pH-Wertes durch Ammoniak oder Amine auszuschließen ist, da diese Substanzen mit der Oxogruppe der zur Azomethinbildung befähigten Verbindungen reagieren würden und damit die Wirkung dieser Verbindung unterbunden würde.

Für die Einstellung des pH-Wertes ist hingegen die Zugabe von Elektrolyten aus der Gruppe Natriumion oder Kaliumion sehr zu empfehlen, wobei im Falle einer festen Zubereitung die Elektrolyte ganz oder teilweise in Form eines Monosalzes der Ketoglutarsäure zugesetzt werden können.

Das Mittel gemäß vorliegender Erfindung wird vorzugsweise als intravenös zu verabreichende Spezialität formuliert, insbesondere in Form einer Infusion.

Mit dieser Darreichungsform kann am verlässlichsten ein gleichmäßig hoher Blutspiegel erzielt werden, durch den die zerstörende Wirkung auf den Tumor voll entfaltet wird. Dies gilt vor allem für solche maligne Erkrankungen, bei denen die Wirkstoffe des erfindungsgemäßen Mittels nur über die Blutbahn herangebracht bzw. in den Tumor eingeschleust werden. Es ist aber möglich, das Mittel für eine orale oder rektale Verabreichung zu formulieren. Diese Form ist dann vorteilhaft, wenn das Mittel über den Verdauungsweg direkt zum Tumor gelangen kann.

Handelt es sich um eine Erkrankung in einer nach außen geöffneten Körperhöhle, kann das erfindungsgemäße Mittel auch als Spülung verabreicht werden.

In der Regel werden aber oral zu verabreichende Formen, wie Kapseln, Tabletten, aber auch Suspensionen oder Lösungen sowie solche Mittel, die rektal verabreicht werden, nicht in der Anfangsphase der Therapie eingesetzt. Diese Therapieformen bieten sich vor allem für die Nachbehandlung von bereits erfolgreich mit dem erfindungsgemäßen Mittel behandelten Patienten an, die bereits in häusliche Pflege entlassen wurden. Ferner können diese Darreichungsformen während einer Infusionstherapie an Tagen in häuslicher Pflege, meistens am Wochenende, vor allem in oraler Form, verabreicht werden, um damit eine Medikationslücke, die den Blutspiegel negativ beeinflussen würde, zu überbrücken.

Für die Formulierung des erfindungsgemäßen Mittels als Infusionslösung hat sich folgende Zusammensetzung sehr bewährt:

| | |
|---|---|
| alpha-Ketoglutarsäure | 3-20 g/l |
| 5-Hydroxymethylfurfural | 1-3 g/l |
| N-Acetyl-seleno-L-methionin | 1,4-2,3 mg/l |
| N-Acetyl-L-methionin | 70-230 mg/l |
| Glucose | 20-100 g/l |
| Natriumion | 60-160 mmol/l |
| Kaliumion | 15-40 mmol/l |

wobei mit einer Zusammensetzung für Infusionen

| | |
|---|---|
| alpha-Ketoglutarsäure | 6,0-16,0 g/l |
| 5-Hydroxymethylfurfural | 1,0-2,5 g/l |
| N-Acetyl-seleno-L-methionin | 1,4-2,3 mg/l |
| N-Acetyl-L-methionin | 70-230 mg/l |
| Glucose | 20-50 g/l |
| Natriumion | 70-160 mmol/l |
| Kaliumion | 20-40 mmol/l |

besonders gute Ergebnisse erzielbar sind. Dies trifft besonders bei Anwendung in der Anfangsphase der Medikation zu. Die in der eigentlichen Behandlungsphase angewandten Tagesdosen sind in der Regel 3-30 g alpha-Ketoglutarsäure,1-5 g 5-Hydroxymethylfurfural, 1,4-2,3 mg N-Acetyl-seleno-L-methionin und etwa das 100-fache an N-Acetyl-L-methionin.

Bei Präparaten, die in fester oder flüssiger Form formuliert werden und oral oder rektal z.B. bei einer Gastrointestinal-Erkrankung verabreicht werden, ist es in Bezug auf den pH-Wert günstig, wenn die Ketoglutarsäure zumindest zum Teil in Form ihrer Mono-Natrium- oder Mono-Kaliumsalze enthalten ist. Bei derartigen Präparaten liegt die Tagesdosis in der Regel etwas niedriger als bei Verabreichung einer Infusion. Sie beträgt mindestens 3-9 g alpha-Ketoglutarsäure und 0,5-1,5 g 5-Hydroxymethylfurfural, 1,4-2,3 mg N-Acetyl-seleno-L-methionin und 70-230 mg N-acetyl-L-methionin enthalten. Der pH-Wert liegt bei einem oral zu verabreichenden Mittel unter 4.

Ferner ist bei oral zu verabreichenden Formen des Präparates ein Zusatz von Glucose oder eines Gemisches von Glucose und Fructose vorteilhaft, besonders günstig ist jedoch die Einverleibung eines Disaccharides, besonders von Rohrzucker, da damit eine Geschmacksverbesserung erreicht wird.

Das Mittel Kann auch verdünnungs-und/oder Streckmittel enthalten.

Für eine Nachbehandlung wird eine noch niedrigere Dosis gewählt, wobei einer Tagesdosis von 2,25 g Ketoglutarsäure und 0,375 g 5-Hydroxymethylfurfural, 1,5-2,0 mg N-Acetyl-seleno-L-methionin und 150-200 mg N-Acetyl-L-methionin der Vorzug gegeben wird.

Zur Herstellung des erfindungsgemäßen Mittels in einer zur intravenösen Verabreichung geeigneten Form wird die alpha-Ketoglutarsäure bei erhöhter Temperatur in destilliertem, durch Begasen sauerstoffarm gemachtem Wasser gelöst, die so erhaltene Lösung nach etwaigem Zusatz von Glucose oder Fructose durch den Zusatz von Alkalien, nicht aber von Ammoniak oder Aminen, auf einen pH-Wert von etwas über 4 eingestellt, und dieser Mischung werden dann das N-Acetyl-seleno-L-methionin, das N-Acetyl-L-methionin und die zur Azomethinbildung befähigte Verbindung zugesetzt. Ist ein Zusatz von Glucose und/oder Fructose vorgesehen, so erfolgt die Zugabe dieser Verbindungen bei Herstellung einer Infusionslösung vor oder gemeinsam mit der Einstellung des pH-Wertes.

Im Falle der Herstellung von Mitteln zur oralen oder rektalen Verabreichung wird zur Einstellung eines pH-Wertes von 3-6 die Ketoglutarsäure entweder zum Teil oder zur Gänze in Form ihres Monosalzes mit Natrium und/oder Kalium eingesetzt und mit Streckmitteln und gewünschtenfalls auch mit Disacchariden gemischt, worauf der Mischung die zur Azomethinbildung befähigte Substanz, das N-Acetyl-seleno-L-methionin und das N-Acetyl-L-methionin zugesetzt werden, worauf die Mischung in die gewünschte Darreichungsform, insbesondere in ein Trinkgranulat, in Tabletten oder in eine Spülung, übergeführt wird. Bei dem Wirkstoff, der zur Azomethinbildung befähigt ist, muss bei dessen Auswahl aus den genannten Substanzen beachtet werden, dass die gewählte Verbindung keinen intensiven oder gar unangenehmen Geschmack besitzt. Auch in dieser Hinsicht ist das 5-Hydroxymethylfurfural die beste Wahl.

### BESTE AUSFÜHRUNGSFORMEN DER ERFINDUNG

In den nachfolgenden Beispielen werden detaillierte Rezepturen für behandlungsgerechte Formen des erfindungsgemäßen Mittels gegeben:

### Beispiel 1

alpha-Ketoglutarsäure 6,000 g/l
5-Hydroxymethylfurfural 2,000 g/l
N-Acetyl-seleno-L-methionin 1,5 mg/l
N-Acetyl-L-methionin 150 mg/l
Glucose 50,000 g/l
KOH 85%ig 1,320 g/l
NaOH 1,200 g/l

Aus diesen Substanzen wird ein Liter einer Lösung bereitet, indem man zunächst die Ketoglutarsäure in destilliertem Wasser bei einer Temperatur von etwa 50°C löst, das vorher durch Begasen sauerstoffarm gemacht wurde. In die resultierende Lösung werden dann nacheinander NaOH und KOH als Elektrolyte sowie die Glucose eingetragen, wobei gleichzeitig ein pH-Wert von etwas über 4 eingestellt wird. Der so erhaltenen klaren Lösung werden schließlich unter Rühren das Hydroxymethylfurfural und die beiden Methioninderivate hinzugefügt. Man erhält so eine klare, leicht gelbliche Lösung, die pro Liter 6 g Ketoglutarsäure, 2 g 5-Hydroxymethylfurfural, 1,5 mg N-Acetyl-seleno-L-methionin und 150 mg N-Acetyl-L-methionin als Wirkstoffe und ferner 50 g Glucose sowie Elektrolyte in folgenden molaren Konzentrationen enthält:
Na⁺30, 00 mmol/l
K⁺ 20,00 mmol/l

Der pH-Wert der Lösung beträgt 4,90. Die Lösung hat eine berechnete Osmolarität von 385 mosmol/l Lösung. Sie wird zur Anwendung als Infusionslösung in Flaschen von 1/2 Liter Inhalt abgefüllt.

### Beispiel 2

alpha-Ketoglutarsäure 16,000 g/l
5-Hydroxymethylfurfural 2,000 g/l
N-Acetyl-seleno-L-methionin 2,3 mg/l
N-Acetyl-L-methionin 200 mg/l
Glucose 20,000 g/l
KOH 85%ig 1,650 g/l
NaOH 4,000 g/l

Wie in Beispiel 1 beschrieben wird aus den o.a. Substanzen 1 Liter einer Lösung bereitet, die neben 16 g Ketoglutarsäure, 2,3 mg N-Acetyl-seleno-L-methionin, 200 mg N-Acetyl-L-methionin, 2 g 5-Hydroxymethylfurfural sowie 20 g Glucose und Elektrolyte in folgender molarer Konzentration enthält:
Na⁺160,00 mmol/l
K⁺ 25,00 mmol/L

Der pH-Wert der Lösung beträgt 4,1. Die berechnete Osmolarität etwa 362 mosmol/l Lösung. Die Lösung wird in Infusionsflaschen von 1/2 Liter Inhalt abgefüllt.

### Beispiel 3

alpha-Ketoglutarsäure 12,000 g/l
5-Hydroxymethylfurfural 2,000 g/l
N-Acetyl-seleno-L-methionin 2,0 mg/l
N-Acetyl-L-methionin 150 mg/l
Glucose 20,000 g/l
KOH 85%ig 1,320 g/l
NaOH 4,000 g/l

Wie in Beispiel 1 beschrieben wird aus diesen Substanzen 1 1 einer Lösung bereitet, die die Wirkstoffe Ketoglutarsäure und 5-Hydroxymethylfurfural im Verhältnis 6:1 enthält. Weiters sind Elekrolyte in folgender molarer Konzentration zugegeben:
Na⁺ 100, 00 mmol/l
K⁺ 20,00 mmol/L

Der pH-Wert der Lösung beträgt 4,68. Sie wird in Infusionsflaschen von 1/2 l Inhalt abgefüllt.

### Beispiel 4

345,12 g alpha-Ketoglutarsäure-mono-Na-Salz, 150 mg N-Acetyl-seleno-L-methionin, 7,5 g N-Acetyl-L-methionin und 1.190 g Staubzucker werden in einem Planetenmischer trocken gemischt und durch ein Sieb der Maschenweite 0,7 mm gesiebt. Das so erhaltene Material wird in den Mischer zurückgegeben und bei laufendem Mischwerk mit 100 g destilliertem Wasser versetzt und bis zur Agglomeratbildung gemischt. Nach Trocknung bei 50°C wird das Produkt durch ein Sieb der Maschenweite 1,25 mm granuliert und in einem Planetenmischer mit 50,0 g 5-Hydroxymethylfurfural gemischt. Der pH-Wert beträgt etwa 3. Man erhält 1.600 g einer Masse, die als Trinkgranulat eingesetzt werden kann. Sie wird in Portionsbeutel zu je 4 g abgefüllt. Dieser Portionsbeutel enthält 0,75 g Ketoglutarsäure, 0,125 g Hydroxymethylfurfural sowie 0,375 mg N-Acetyl-seleno-L-methionin und 18,75 mg N-Acetyl-L-methionin.

### Beispiel 5

Die gleichen Stoffe wie in Beispiel 4, jedoch mit dem Unterschied, dass anstelle der Saccharose übliche Gleit- und Sprengmittel eingesetzt werden, werden zu Tabletten verarbeitet und anschließend mit einem magensaftresistenten Überzug versehen. Sie können zur Behandlung des Dünndarms eingesetzt werden.

### Beispiel 6

15 g Methylzellulose, die in Form eines flüssigen Schleims vorliegt, werden mit
alpha-Ketoglutarsäure 6,000 g/l
N-Acetyl-seleno-L-methionin 1,5 mg/l
N-Acetyl-L-methionin 75 mg/l
KOH 85%ig 0,726 g/l
NaOH 1,200 g/l
NaH₂PO₄.2H₂O 16,00 g/l
Na₂HPO₄**.**12H₂O 6,00 g/l
5-Hydroxymethylfurfural 1,00 g/l
vermischt. Die resultierende Lösung einer Viskosität von 20-50 mPa.s und einem pH-Wert von etwa 6 wird auf einen Liter aufgefüllt. Sie kann in Portionen von 1/4 Liter als Klistier verabreicht werden und dient bevorzugt zur Behandlung von Coloncarcinomen, wobei die Tagesdosis 2 Klistiere beträgt.

Mit den erfindungsgemäßen Mitteln zur Bekämpfung von Krebs können prinzipiell alle bösartigen Tumorerkrankungen, die mit einer Erhöhung der alpha-Ketoglutarsäure im Serum verbunden sind, behandelt werden.

Die Anwendung des erfindungsgemäßen Mittels in der Therapie von Patienten mit malignen Tumoren an Organen wie Lunge, Bronchien, Brust, Blase, Magen und dergleichen, die die oben genannte Forderung eines erhöhten Blutspiegels an Ketoglutarsäure erfüllt hatten, führte bei einer Behandlung von einigen Wochen in den meisten Fällen zu positiven Ergebnissen. So konnte nach einem Behandlungszeitraum von 1-2 Monaten in mehreren Fällen ein Stadium erreicht werden, in dem der zuvor deutlich sichtbare Tumor im Röntgenbild nicht mehr nachweisbar war und auch eventuell vorhandene Metastasen erfolgreich bekämpft waren.

Festzuhalten ist allerdings, dass die Medikation unbedingt weiter fortgesetzt werden muss, um einen Rückfall, der dann schwer zu bekämpfen ist, zu vermeiden. Es empfiehlt sich jedoch, im Rahmen der Nachbehandlung die täglichen Dosen, die zu Beginn 3-30 g Ketoglutarsäure und 1-5 g 5-Hydroxymethylfurfural betrug, auf 3-9 g Ketoglutarsäure und 0,5-1,5 g 5-Hydroxymethyfurfural abzusenken.

Der sich mit den erfindungsgemäßen Mitteln einstellende Behandlungserfolg äußerte sich auch in einer entscheidenden Verbesserung des Allgemeinzustandes.

Die Dauer der Behandlung ist unterschiedlich, je nachdem, wie weit das Krebsgeschehen bereits fortgeschritten ist. Auffallend rasche Behandlungserfolge wurden vor allem dort registriert, wo sich das Krebsgeschehen noch in einem frühen Stadium befand. Schädliche oder auch nur unangenehme Nebenwirkungen des erfindungsgemäßen Mittels konnten nicht beobachtet werden.

**I.) Fallbeispiele mit erfindungsgemäßer Behandlung Zusammensetzung der eingesetzten Infusionslösung:**

| | |
|---|---|
| alpha-Ketoglutarsäure | 9,0 g/l |
| 5-Hydroxymethylfurfural | 3,0 g/l |
| N-Acetyl-seleno-L-methionin | 2,0 mg/l |
| N-Acetyl-L-methionin | 100,0 mg/l |
| Glucose | 30,0 g/l |

| | |
|---|---|
| Na⁺ und K⁺ zur pH-Einstellung | |

Die Infusionslösung wurde in Einheiten zu 0,5 l abgefüllt.

Alle Patienten wurden auf folgende Weise behandelt:

Zu Beginn der Infusionsbehandlung bekommen die Patienten die ersten 3 Tage jeweils eine Infusion zu 0,5 l. Ab dem 4. Tag werden dann zwei Infusionen zu 0,5 l verabreicht, wobei jede Infusion in allen Fällen 3 Stunden andauert. Diese Art der Verabreichung wird bis zum Ende der Infusionstherapie eingehalten.

Zum Ausgangsbefund bei Aufnahme und zum Befund nach Therapie wurde jeweils ein Computertomogramm erstellt, wenn verfügbar, wurden Tumormarker bestimmt. In besonderen Fällen (Ösophaguskarzinom) wurde die Nachkontrolle mittels Endoskopie durchgeführt.

### Fall 1: M. G., weiblich:

Die Patientin hatte sich 1975 wegen eines Mammakarzinoms einer Operation unterziehen müssen und erlitt ab 2001 Zeichen eines Rückfalls, der im Rahmen einer Pleurabiopsie (Mammakarzinom-Rezidiv) und bald darauf in einer Entdeckung von multiplen Knochenmetastasen im Stammskelett und Lebermetastasen 2,1-1,1 cm zum Ausdruck kam. Diese Symptome wurden mit einer Hormontherapie behandelt, die Erkrankung entwickelte sich jedoch weiter, und es wurden im Juni 2002 eine Lymphangiosis carcinomatosa sowie eine Lungenembolie diagnostiziert, die von Juni bis Oktober 2002 mit einer Polychemotherapie mit Epirubicin und Taxotere behandelt wurden. Diese Behandlung führte jedoch zu keiner Regredienz der Erkrankung.

Im November 2002 kam die Patientin, die inzwischen als austherapiert bezeichnet wurde, zu Herrn OA Dr. Ralph Herwig, der die Patientin genau untersuchte. Dabei wurden die Lebermetastase an der Leberkuppe und die Rippenmetastasen an der 7. und 9. Rippe rechts bestätigt. Auch die Lymphangiosis carcinomatosa war noch nachweisbar. Die Erkrankung war im Vergleich zu den Vorbefunden progredient (Befundberichte Dr. E. Partl, Krankenhaus Kitzbühel, Labor Dr. Schmoigl, Telfs). Ein Zeichen dafür war u.a. der Tumormarker CA 15-3 55,4 U/ml am 20.11.2002, der bis 19.12.2002 auf 102,1 U/ml angestiegen war.

Am 09.01.2003 wurde mit der Infusionstherapie begonnen. Die tägliche Behandlung wurde bis einschließlich 07.02.2003 verabreicht.

Bei der Aufnahme eines in der Folge angefertigten Computertomogrammes (CT) wurden keine Hinweise auf hepatische Metastasen, keine Hinweise auf Lungenmetastasen, keine Hinweise auf Rippenmetastasen und kein sicherer Hinweis für aktive Lymphangiosis carcinomatosa festgestellt. Der Tumormarker CA 15-3, der am 19.12.2002 auf 102,1 angestiegen war, war am 04.02.2003 auf 35 U/ml abgesunken. Es bestanden keine Nebenwirkungen, eine Schmerzmedikation war nicht nötig, die Mobilität der Patientin war vollständig.

Beurteilung: Regrediente Tumorerkrankung, ohne Hinweise auf Residualtumor in den untersuchten Bereichen.

In der Folgezeit wurde eine Hirnmetastase festgestellt, die auswärts mittels Strahlentherapie behandelt wurde.

### Fall 2: H. I., 50, weiblich:

Niedrig differenziertes Uterus-Ca, G III, Nₓ, Erstdiagnose Februar 2000, anschließend abdominale Hysterektomie und Adnexektomie im Juni 2000. Ab Jänner 2002 begann eine Bestrahlungstherapie, dabei wurden der Reihe nach verschiedene Regionen behandelt und ausbestrahlt: ab Jänner 2002 Bestrahlung des Kreuzbeines, ab Juni 2002 Bestrahlung des Brustwirbelkörpers 12 (30 Gy), ab Oktober 2002 Bestrahlung der rechten Schulter und ab Februar Bestrahlung des Brustwirbelkörpers 10. Im Mai 2003 wurden multiple Lungenmetastasen festgestellt. Die Bestrahlung wurde daher wegen des schlechten Allgemeinempfindens abgebrochen, und am 09.06.2003 wurde die Behandlung mit der erfindungsgemäßen Infusion aufgenommen. Zum Zeitpunkt der Aufnahme stellte man zahlreiche intrapulmonale Metastasen zwischen 5 und 13,5 mm, eine Lebermetastase von 14,5 cm Größe, eine Knochenmetastase im Brustwirbelkörper 12 (2,4 cm), im Lendenwirbelkörper 5 rechts (2,7 cm) sowie Halslymphknoten fest. Ferner benötigte die Patientin bei Aufnahme Mundidol 90 mg, 3x1, Viox 25 mg, 2x1, und Vendal-Saft 8 ml bei Bedarf als ständige Schmerzmedikation. Bei Aufnahme war nur ein liegender Transport möglich, sitzen konnte die Patientin nur 10 Minuten ohne Schmerzen.

Therapiebeginn war am 09.06.2003. Nach Therapie: palpatorisch deutlich regrediente Halslymphknoten rechts, sonografische Regredienz der Lebermetastasen (Markerläsion auf 7,9x5,1 cm geschrumpft). Mobilität war bei Entlassung vollständig gegeben, alle Tätigkeiten waren selbständig durchführbar, die Patientin konnte täglich 3 Stunden ausgehen, insbesondere selbständig einkaufen, auch ein Besuch beim Friseur war möglich.

Schmerzmedikation bei Entlassung: Mundidol 60 mg, 3x1, Vendal-Saft bei Bedarf, 2 ml. Nebenwirkungen traten keine auf.

### Fall 3: F. J., 66, männlich:

Der Patient litt an einem Ösophagus-Ca (eT3, cN1, Mx, GIII), außerdem hatte der Patient einen schwer therapierbaren Diabetes Mellitus Typ II und eine arterielle Hypertonie. Beim Ausgangsbefund wurde ein stenosierendes Ösophaguskarzinom zwischen 30 und 35 cm ab der Zahnreihe festgestellt. Es handelt sich um ein Karzinom in einem Barett-Ösophagus, es bestand Gewichtsverlust von 10 kg, der Patient hatte subjektiv Krankheitsgefühl. Die Mobilität bei der Aufnahme war vollständig. Keine Schmerztherapie bei der Aufnahme.

Am 13.02.2003 wurde mit der Therapie begonnen. Therapieende des ersten Zyklus war am 13.03.2003. Bei der Untersuchung des Patienten nach der Therapie wurde gastroskopisch eine deutliche Abflachung des zuvor exulcerierend wachsenden Karzinoms festgestellt. Es bestand eine deutliche Gewichtszunahme, subjektiv fühlte sich der Patient sehr gut. Die Mobilität bei der Entlassung war vollständig, eine Schmerzmedikation bei der Entlassung war nicht nötig, Nebenwirkungen waren keine festzustellen.

Beurteilung: Regredienz der Erkrankung, Verbesserung des Allgemeinzustandes, subjektiv deutliche Besserung des Allgemeinbefindens. Nach dem 13.03.2003 wurde der Patient mit einer alpha-Ketoglutarsäure/5-Hydroxymethylfurfural-Trinklösung weiter behandelt.

Ein weiterer Behandlungszyklus wurde vom 12.05.2003 bis zum 17.05.2003 durchgeführt. Zusätzlich wurden 1200 mg 5-Fluorouracil verabreicht.

Beurteilung: Bei einer neuerlichen Kontrolle mittels Endoskopie zeigte sich eine weitere Regredienz des Befundes von intraluminal gesehen. Der Befund war nicht mehr stenosierend, der Patient besaß keine Schluckbeschwerden mehr. Das Körpergewicht des Patienten war wieder auf dem Ausgangswert vor dem Gewichtsverlust. Eine histologische Zwischenkontrolle ergab ein Down-Staging auf G II-III, endosonografisch war der Befund auf eT2-3 regredient, die zuvor im CT gesehene Lymphknotenmetastase war nicht mehr nachweisbar. Interessanterweise kam es zu keinem Zeitpunkt zu einer Knochenmarksdeprivation durch die zusätzlich verabreichte Chemotherapie.

### Fall 4: B. E., 76, weiblich:

Die Patientin litt an einem Blasenkarzinom (pT4, N2 = ausgedehnter Lymphknotenbefall, M 1 = Fernmetastasen, G III) mit Stauungsniere rechts. Die Patientin wünschte ausdrücklich die oben genannte Therapie. Eine Blasenbiopsie zur Histologiegewinnung am 04.02.2003 an der Universitätsklinik in Innsbruck ergab ein muskelinvasives Blasenkarzinom mindestens pT2 mit Carcinoma in situ, N2, Mx, GIII. Wegen einer Hydronephrose rechts (tumorbedingte Nierenstauung) musste eine Nierenableitung (Splint) am 17.03.2003 (Universitätsklinik Innsbruck) eingelegt werden.

Am 19.03.2003 kam sie zur Infusionsbehandlung. Ausgangsbefund bei Aufnahme: Es wurden cytoskopisch deutliche Tumormassen, welche in die Harnblase vorwuchsen, festgestellt, der Splint rechts liegt in situ. Das Ostium rechts (Harnleiteröffnung in der Blasenwand) ist nicht eindeutig zu verifizieren. Es wurde sonografisch weiter beobachtet, dass der Tumor mit einer Größe von 3,5x2 cm im Bereich des Ostiums auszumessen ist. Es besteht auch eine Infiltration der Gebärmutter und eine fragliche Infiltration des Mastdarms. Zusätzlich findet sich eine Raumforderung links von der Mittellinie mit 5,3 cm Ausmessung. Bei Aufnahme keine Schmerztherapie, Mobilität bei der Aufnahme vollständig.

Am 19.03.2003 wurde mit der Infusionstherapie begonnen. Die erfindungsgemäße Infusion wurde vom 19.03.2003 bis 06.04.2003 und vom 08.04.2003 bis 20.04.2003 verabreicht. Nach der Therapie zeigte eine cytoskopische Untersuchung des Tumors eine deutliche Regredienz, die Harnleiteröffnung war deutlich sichtbar, die übrige Blasenwand unauffällig. Sonografisch wurde eine Regredienz der Raumforderung im Ostiumbereich festgestellt. Eine Biopsie eines Lymphknotens in der linken Leiste ergab sklerotisches Bindegewebe mit herdförmiger Infiltration durch solide oder einzellige, infiltrierende, polymorphe Zellkomplexe des vorbeschriebenen, gering differenzierten Blasenkarzinoms. Die Sklersoierung von Gewebe im Lymphknoten findet sich sonst nur als Zeichen der Tumorreduktion nach Chemotherapie. In einer CT-Untersuchung vom 02.05.2003 (Universität Innsbruck) konnte eine deutliche Regredienz einer nodulären (Lymphknoten) Tumormanifestation von 5,5 auf 4,9 cm gesichert werden. Die Harnblasenwand war nur im Rahmen der Messgenauigkeit etwas prominenter. Eine neu aufgetretene Lymphknotenvergrößerung paraaortal und interaortocaval (maximal 1,7 cm messend) ist nur im Verlauf zu beurteilen. Die Regredienz der Tumormanifestation im Ostiumbereich ist auch hier nachzuweisen. Die weiteren Untersuchungen ergaben keinen Anhalt für Knochenmetastasierung. Insgesamt findet sich auch in dieser CT eine Regredienz der Erkrankung. Mobilität war bei Entlassung vollständig gegeben. Keine Schmerzmedikation bei Entlassung, keine Nebenwirkungen.

Beurteilung: regredienter Befund, histologischer Nachweis der Lymphknotensklerose (Vernarbung).

### Fall 5: O. H., 65, weiblich:

Bei der Erkrankung dieser Patientin handelt es sich um ein metastasiertes Mammakarzinom mit Lebermetastasen, Lungenmetastasen, malignem Pleuraerguss, Aszites, Leukopenie, Thrombopenie und Gerinnungsstörung bei beginnendem Leberversagen.

Nach mehrfachen Polychemotherapien traten kurz vor Therapiebeginn mit der erfindungsgemäßen Infusionslösung eine therapieresistente Aplasie und Gerinnungsstörungen auf. Eine Sauerstoffsubstitution (4 1/min) bei respiratorischer Insuffizienz nach dem letzten Chemotherapiezyklus war notwendig. Aszites und Pleuraerguss waren wegen der Aplasie nicht punktierbar. Die Patientin war immobil und bettlägerig.

Vom 07.02.2003 bis 06.03.2003 wurde die Behandlung mit der erfindungsgemäßen Infusionslösung durchgeführt. Anschließend wurde die Patientin mit einer alpha-Ketoglutarsäure/5-Hydroxymethylfurfural-Trinklösung oral nachbehandelt. Danach waren die vorbeschriebenen Lebermetastasen nicht mehr nachweisbar (Sonografie des Krankenhauses Kitzbühel). Der Pleuraerguss und der Aszites waren deutlich rückläufig und nicht mehr punktionswürdig, die Sauerstoffsubstitution war ebenfalls nicht mehr notwendig. Radiologisch zeigte sich eine deutliche Besserung der pulmonalen Situation (Befunde Dr. E. Partl, Krankenhaus Kitzbühel). Ein schneller Anstieg der hämatologischen Parameter inkl. raschem Thrombozytenanstieg war nachweisbar. Die Patientin ist vollständig mobil aus dem Krankenhaus entlassen worden.

### Fall 6: P. P., 62, männlich:

Metastasierendes Bronchialkarzinom, multiple Lungen- und Lymphknotenmetastasen, Lungenfunktionseinschränkung.

Bei der Aufnahme deutliche respiratorische Insuffiz, keine Schmerztherapie, vollständige Mobilität.

Therapie mit der erfindungsgemäßen Infusionslösung vom 13.05.2003 bis 13.06.2003. Danach Regredienz der Raumforderung (der Lymphknotenmetastase) rechts, axillär, die übrigen Tumormassen sind stabil. Deutliche Besserung des Allgemeinzustandes, Verbesserung der Belastbarkeit (ausgedehnte Spaziergänge und Schwimmen möglich). Mobilität bei Entlassung vollständig, keine Schmerzmedikation, keine Nebenwirkungen. Die Tumormassen zeigen insgesamt eine ca. 50%-ige Regredienz (CT Dr. E. Partl, Krankenhaus Kitzbühel).

Beurteilung: regredienter Befund, Verbesserung des Allgemeinzustandes, subjektiv deutliche Besserung des Allgemeinbefindens.

**II. Fallbeispiele mit einer Behandlung gemäß EP 326.826 B1 Zusammensetzung der eingesetzten Mittel:**

| a) Infusionslösung mit einem Volumen von 0,5 l, enthaltend | |
|---|---|
| alpha-Ketoglutarsäure | 3,0 g |
| 5-Hydroxymethylfurfural | 1,0 g |
| Glucose | 25,0 g |
| Natrium-Ionen | 35,0 mmol |
| Kalium-Ionen | 10,0 mmol |
| Calcium-Ionen | 4,0 mmol |
| Magnesium-Ionen | 2,0 mmol |
| Zink-Ionen | 0,0365 mmol |
| Phosphat-Ionen | 20,0 mmol |
| Chlorid-Ionen | 8,0 mmol |

| b) Infusionslösung mit einem Volumen von 1 l, enthaltend | |
|---|---|
| alpha-Ketoglutarsäure | 6,0 g |
| 5-Hydroxymethylfurfural | 2,0 g |
| Glucose | 50,0 g |
| Natrium-Ionen | 70,0 mmol |
| Kalium-Ionen | 20,0 mmol |
| Calcium-Ionen | 8,0 mmol |
| Magnesium-Ionen | 4,0 mmol |
| Zink-Ionen | 0,073 mmol |
| Phosphat-Ionen | 20,0 mmol |
| Chlorid-Ionen | 16,1 mmol |

| c) Präparat für die orale Verabreichung Trinkgranulat, ein Portionsbeutel, enthaltend | |
|---|---|
| alpha-Ketoglutarsäure | 0,75 g |
| 5-Hydroxymethylfurfural | 0,125 g |
| ZnO | 5,0 mg |
| Staubzucker | 3,007 g |

Die Patienten erhielten einmal täglich eine Infusion zu 1 l oder 0,5 l. Die Verabreichung erfolgte täglich, die Wochenenden, an denen keine Infusionen verabreicht wurden, wurden durch das orale Präparat ersetzt.

### Fall 7: H. C., weiblich, 1908:

Diagnose: Karzinom der rechten Brust, 1986 palliative Mastektomie einschließlich Entfernung von Muskeln der Brustwand. Bei einer Untersuchung am 16.01.1987 wurde in der rechten Achsel ein geschwollener Lymphknoten festgestellt.

Nach dieser Diagnose wendete sich die Patientin an jenen Arzt, der im Rahmen einer Versuchsreihe die Infusionslösungen gemäß EP 326.826 B1 an austherapierten Patienten überprüfte. Da diese Patientin offensichtlich zunehmend schwächer wurde, ein Gewichtsverlust auftrat und sie bereits bettlägerig war, wurde sie ab 18.05.1987 pro Tag mit 0,5 l der Versuchsinfusion a) behandelt. Nach 6 Infusionen konnte sie wieder aufstehen, und die Größe der Lymphknoten war reduziert. Nach insgesamt 14 Infusionen wurde die Patientin auf eine Weiterbehandlung mit dem oralen Präparat c) umgestellt. Das Präparat wurde 3x täglich, 6 Monate lang, vorgeschrieben.

Am 04.12.1987 wurde die Patientin radiologisch untersucht. Es wurde sowohl im Lungenbereich als auch in der Achselhöhle kein vergrößerter Lymphknoten mehr bemerkt. Es war also ab diesem Zeitpunkt mit einer guten Prognose zu rechnen.

Im Jahr 1988 traten jedoch zwei kleine Herde in der Operationsnarbe auf. Diese Herde wurden im gleichen Jahr durch eine Operation entfernt. Am 12.05.1989 wurde ein Tumor an der noch vorhandenen linken Brust festgestellt. Dieser wurde am 20.05.1989 entfernt, und es wurde festgestellt, dass er benign ist. Es gab keinerlei Spuren von bösartigen Erscheinungen mehr.

### Fall 8: B. L., weiblich, 1912:

Bei dieser Patientin wurde ebenfalls ein Brustkrebs mit Lymphoma diagnostiziert. Es wurden aber zusätzlich auch Lebermetastasen festgestellt.

Am 31.03.1989 wurde mit der Infusionsbehandlung mit der Versuchsinfusion b) begonnen. Diese Behandlung führte zu einer Schmerzlinderung, gleichzeitig entwickelte sich aber eine zunehmend stärker werdende Gelbsucht, sodass die Behandlung am 14.04.1989 abgebrochen wurde. Die Patientin ist am 29.04.1989 verstorben.

### Fall 9: H. F., männlich, 1926:

Der Patient hatte ein invasives ductales Mammakarzinom rechts (pT 4 BG? M0, N0) und eine modifizierte, radikale Mastektomie rechts sowie axilläre Lymphadenektomie am 13.02.1987 hinter sich. Eine ergänzende Sonographie des Oberbauches und eine Ganzkörperskelettszintigraphie erbrachten keine Hinweise auf Metastasierung.

Wegen des total fortgeschrittenen Geschehens wurde der Patient einer Thoraxwandbestrahlung unterworfen, zu der der Patient nach der 5. Bestrahlung nicht mehr erschien. Für den Lymphknoten bestand eine günstige Prognose (04.04.1987). In einer Röntgenuntersuchung vom 18.08.1987 war ein ausgedehnter Erguss im rechten Pleuraraum sichtbar, der zu Schmerzen führte. Am 29.09.1987 wurde erstmals ein Verdacht auf Skelettmetastasen geäußert, die dann zu Schmerzen im rechten Brustraum, Schmerzen im Bereich der Lendenwirbelsäule und der linken Hüfte führten.

Am 03.10.1987 wurde mit der Infusionstherapie mit Infusionslösung a) begonnen, zunächst in der Wohnung, dann ab 05.10.1987 täglich in der Ordination. Am 07.10.1987 wurde Metastasenbefall der Lendenwirbelsäule bestätigt. Man fand multiple, kleinste osteolytische Destruktionsherde in nahezu allen Lendenwirbelkörpern. Weitere multiple, kleinfleckige osteolytische Destruktionsherde im Bereich beider Femurköpfe sowie Schenkelhälse. Die Infusionen wurden trotzdem weitergegeben. Diese gaben ihm das allgemein Fehlende zur Wiederbelebung des Stoffwechsels und Änderung des Allgemeinempfindens. Bis zum 19.10. bekam er 11 Infusionen. Dann wurde die Therapie wegen Verwirrtheitszuständen und der Gefahr von Spontanfrakturen eingestellt. Am 26.10. wurde das Vorhandensein von Gehirnmetastasen bestätigt. Am 30.10.1987 verstarb der Patient.

### Fall 10: K. R., männlich, 1925:

Der Patient hatte ein inoperables Bronchialkarzinom im rechten Unterlappen, Probatoria, massiven Lymphknotenbefall im gesamten Mediastinum (Mittelfell) und Infiltration in das Pericard (Herzbeutel) und in die Speiseröhre (Oesophagus). Der Patient kam von der Thoraxstation.

Infusionsbeginn: 11.08.1987 mit Infusionslösung b), BSG 75/115, 3,0 Mio Ery und 10,5 Hb. Es bestanden beim Patienten multiloculäre Schmerzen, er bekam Temgesic. Auch Muskelkrämpfe machten ihm zu schaffen.

Am 24.08., nach der 8. Infusion, ging es dem Patienten nach seiner Aussage super. Ob sich die diversen Metastasen zurückzubilden begannen, wurde nicht untersucht, da nach der bisherigen Kürze der Therapie noch kaum eine Rückbildung erwartet werden konnte. Die Infusionen wurden fortgesetzt, bis zum 07.09.1987, das sind 19 Infusionen. Auf Wunsch der Angehörigen wurde die Infusionstherapie abgebrochen und der Patient in das Krankenhaus der Barmherzigen Brüder gebracht, wo er bereits am 23.09.1987 verschied. Das spricht dafür, dass sich durch die Infusionstherapie das Allgemeinbefinden verbessert hat, aber der massive Befall der Umgebung des Krankheitsherdes, nämlich der Lymphknoten und des Mediastinums, kaum oder gar nicht angesprochen hatte, als die Infusionstherapie abgebrochen wurde.

### Diskussion der Fallbeispiele:

Die 6 Fallbeispiele des Teiles I ergaben in allen Fällen einen erstaunlichen Erfolg, da es sich ausschließlich um austherapierte Patienten handelte und daher ein fortgeschrittener Prozess mit häufigen, verschiedenen Metastasen vorlag.

Betrachtet man z. B. den Fall 1 einer Patientin, die schon 1975 wegen eines Mammakarzinoms operiert worden war und ab 2002 Zeichen eines Rückfalles in Form einer Pleuritis carcinomatosa zeigte, der dann sehr rasch das Auftreten von Lebermetastasen und ossären Metastasen folgte, die durch keine Chemotherapie bekämpft werden konnten, war der Fall dieser Patientin als sehr ernst anzusehen. Alle diese bereits massiven Merkmale der fortgeschrittenen Erkrankung wurden jedoch durch die 1 Monat dauernde Infusionsbehandlung gemäß der vorliegenden Erfindung so intensiv bekämpft, dass bei Aufnahme eines Tomogrammes im Rahmen der Untersuchung nach Therapie nicht nur ein Befall der Lymphknoten, sondern sowohl die Lebermetastasen als auch die multiplen Knochenmetastasen im Stammskelett nicht mehr feststellbar waren. Dementsprechend waren auch die Mobilität und das Allgemeinbefinden sehr gut. Diese Patientin wäre jedoch mit einer Therapie gemäß der EP 326.826 B1 nicht zu retten gewesen, weil diese Lösung weder Leber- noch Knochenmetastasen angreift (siehe dazu Fall 8).

Die gleiche Situation ist auch im Falle von Knochenmetastasen, die durch die Infusion gemäß EP 326.826 B1 nicht angegriffen werden, vorhanden. Das sieht man deutlich im Fall 9, wo man längere Zeit der Meinung war, dass keine Knochenmetastasen vorliegen. Dadurch wurde auch eine Infusionsbehandlung erst begonnen, als man vermutete, dass doch Knochenmetastasen gebildet worden sind. Die Infusionsbehandlung wurde 4 Tage vor der Diagnose, dass in der Lendenwirbelsäule Knochenmetastasen vorhanden sind, begonnen. Trotz der weiter durchgeführten Infusionstherapie breiteten sich die Knochenmetastasen sehr rasch weiter aus. Erst 7 Tage vor dem Tod des Patienten wurde die Infusionstherapie eingestellt. Der behandelnde Arzt hatte diese Therapie nur verlängert, damit das Allgemeinempfinden besser wird. Dass die Knochenmetastasen nicht bekämpft werden können, war ihm offenbar klar.

Die Tatsache, dass mit der erfindungsgemäßen Lösung Leber- und Knochenmetastasen bekämpft und sogar entfernt werden können, was für die bekannte Lösung nicht zutrifft, beschränkt sich nicht auf das Mammakarzinom, wie im Fall 6 an Bronchialkrebs gezeigt worden ist. Im Vergleich dazu konnte im Fall 10 am Patienten, der auch ein Bronchialkarzinom hatte, gezeigt werden, dass auch in diesem Fall die Infusionstherapie gemäß EP 326.826 B1 nur das Allgemeinbefinden verbesserte, den massiven Befall der Umgebung des Krankheitsherdes aber nicht aufhalten oder gar beseitigen konnte.

## Patentansprüche

1. Mittel mit zerstörender Wirkung auf maligne Tumore, das als Wirkstoffe alpha-Ketoglutarsäure oder deren pharmazeutisch verträgliche Salze und mindestens eine in enzymunabhängiger Reaktion zur Azomethinbildung befähigte Verbindung aus der Gruppe 5-Hydroxymethylfurfural, Dehydroascorbinsäure, Maltol und Vanillin umfasst, wobei vorzugsweise das Masseverhältnis der Ketoglutarsäure zu der mindestens einen zur Azomethinbildung befähigten Verbindung größer als 1:1, insbesondere 2:1 bis 12:1, ist, **dadurch gekennzeichnet, dass** das Mittel als weitere wirkstoffe N-Acetyl-seleno-L-methionin und N-Acetyl-L-methionin enthält, wobei Letzteres im Überschuss gegenüber Ersterem vorliegt.

2. Mittel nach Anspruch 1, **dadurch gekennzeichnet, dass** das Masseverhältnis von alpha-Ketoglutarsäure zu N-Acetyl-seleno-L-methionin 100:1 bis 20000:1, vorzugsweise 500:1 bis 10000:1, beträgt.

3. Mittel nach Anspruch 1 oder 2, **dadurch gekennzeichnet, dass** das Masseverhältnis von N-Acetyl-L-methionin zu N-Acetyl-seleno-L-methionin 20:1 bis 300:1, vorzugsweise 50:1 bis 100:1 beträgt.

4. Mittel nach einem der Ansprüche 1 bis 3, **dadurch gekennzeichnet, dass** es zusätzlich Glucose, Fructose oder ein Gemisch derselben enthält.

5. Mittel nach einem der Ansprüche 1 bis 4, **dadurch gekennzeichnet, dass** die zur Azomethinbildung befähigte Verbindung 5-Hydroxymethylfurfural ist.

6. Mittel nach einem der Ansprüche 1 bis 5, **dadurch gekennzeichnet, dass** es in wässriger Lösung vorliegt und dass das N-Acetyl-seleno-L-methionin in einer Menge von 1,4-2,3 mg/l und das N-Acetyl-L-methionin in einer Menge von 70-230 mg/l vorliegt.

7. Mittel nach einem der Ansprüche 1 bis 6, **dadurch gekennzeichnet, dass** es Elektrolyte aus der Gruppe Natrium oder Kalium enthält.

8. Mittel nach einem der Ansprüche 1 bis 7, **dadurch gekennzeichnet, dass** es intravenös verabreichbar ist und dass der pH-Wert 4-6 beträgt.

9. Mittel nach den Ansprüchen 4, 5, 6, 7 und 8, **dadurch gekennzeichnet, dass** alpha-Ketoglutarsäure in einer Konzentration von 3-20 g/l, 5-Hydroxymethylfurfural in einer Konzentration von 1-3 g/l, Glucose in einer Konzentration von 20-100 g/l, Natriumionen in einer Konzentration von 60-160 mmol/l und Kaliumionen in einer Konzentration von 15-40 mmol/l vorliegt.

10. Mittel nach Anspruch 9, **dadurch gekennzeichnet, dass** alpha-Ketoglutarsäure in einer Konzentration von 6-16 g/l, 5-Hydroxymethylfurfural in einer Konzentration von 1-2,5 g/l, Glucose in einer Konzentration von 20-50 g/l, Natriumionen in einer Konzentration von 70-160 mmol/l und Kaliumionen in einer Konzentration von 20-40 mmol/l vorliegt.

11. Mittel nach einem der Ansprüche 1 bis 5 oder 7, **dadurch gekennzeichnet, dass** es in fester oder flüssiger, oral oder rektal zu verabreichender Darreichungsform vorliegt, die die Ketoglutarsäure zumindest zum Teil in Form ihrer Mono-Natrium- oder Mono-Kaliumsalze enthält.

12. Mittel nach Anspruch 11, **dadurch gekennzeichnet, dass** es Verdünnungs- und/oder Streckmittel und/oder zur Geschmacksverbesserung Disaccharide, insbesondere Staubzucker, enthält.

13. Mittel nach Anspruch 11 oder 12, **dadurch gekennzeichnet, dass** die Dosierungseinheiten 3-9 g alpha-Ketoglutarsäure, 0,5-1,5 g 5-Hydroxymethylfurfural, 1,4-2,3 mg N-Acetyl-seleno-L-methionin und 70-230 mg N-acetyl-L-methionin enthalten.

14. Verfahren zur Herstellung eines Mittels in einer zur intravenösen Verabreichung geeigneten Form nach einem der Ansprüche 8 bis 10, **dadurch gekennzeichnet, dass** die alpha-Ketoglutarsäure bei erhöhter Temperatur in destilliertem, durch Begasen sauerstoffarm gemachtem Wasser gelöst wird, die so erhaltene Lösung nach etwaigem Zusatz von Glucose oder Fructose durch den Zusatz von Alkalien, nicht aber von Ammoniak oder Aminen, auf einen pH-Wert von etwas über 4 eingestellt wird, und dieser Mischung dann das N-Acetyl-seleno-L-methionin, das N-Acetyl-L-methionin und die zur Azomethinbildung befähigte Verbindung zugesetzt werden.

15. Verfahren zur Herstellung von zur oralen oder rektalen Verabreichung geeigneten Präparaten nach einem der Ansprüche 11 bis 13, **dadurch gekennzeichnet, dass** zur Einstellung eines pH-Wertes von 3-6 die Ketoglutarsäure entweder zum Teil oder zur Gänze in Form ihres Monosalzes mit Natrium und/oder Kalium eingesetzt und mit Streckmitteln und gewünschtenfalls auch mit Disacchariden gemischt wird, worauf der Mischung die zur Azomethinbildung befähigte Substanz, das N-Acetyl-seleno-L-methionin und das N-Acetyl-L-methionin zugesetzt werden, worauf die Mischung in die gewünschte Darreichungsform, insbesondere in ein Trinkgranulat, in Tabletten oder in eine Spülung, übergeführt wird.

16. Mittel gemäß einem der Ansprüche 1 bis 13 als Arzneimittel.

17. Verwendung der in den Ansprüchen 1-11 genannten Stoffe zur Herstellung eines Arzneimittels gegen maligne Tumore.

## Claims

1. Agent having a destructive effect on malignant tumours, which comprises, as active ingredients, alpha-ketoglutaric acid or its pharmaceutically compatible salts and at least one compound that is capable of forming azomethine in an enzyme-independent reaction, from the group 5-hydroxymethylfurfural, dehydroascorbic acid, maltol and vanillin, the mass ratio of the ketoglutaric acid to the at least one compound that is capable of forming azomethine preferably being greater than 1:1, in particular 2:1 to 12:1, **characterised in that** the agent contains, as further active ingredients, N-acetyl-seleno-L-methionine and N-acetyl-L-methionine, the latter being present in excess compared to the former.

2. Agent according to claim 1, **characterised in that** the mass ratio of alpha-ketoglutaric acid to N-acetyl-seleno-L-methionine is 100:1 to 20,000:1, preferably 500:1 to 10,000:1.

3. Agent according to claim 1 or 2, **characterised in that** the mass ratio of N-acetyl-L-methionine to N-acetyl-seleno-L-methionine is 20:1 to 300:1, preferably 50:1 to 100:1.

4. Agent according to any one of claims 1 to 3, **characterised in that** it also contains glucose, fructose or a mixture thereof.

5. Agent according to any one of claims 1 to 4, **characterised in that** the compound that is capable of forming azomethine is 5-hydroxymethylfurfural.

6. Agent according to any one of claims 1 to 5, **characterised in that** it is present in an aqueous solution and **in that** the N-acetyl-seleno-L-methionine is present in a quantity of 1.4-2.3 mg/l and the N-acetyl-L-methionine is present in a quantity of 70-230 mg/l.

7. Agent according to any one of claims 1 to 6, **characterised in that** it contains electrolytes of the sodium or potassium group.

8. Agent according to any one of claims 1 to 7, **characterised in that** it can be administered intravenously and the pH is 4-6.

9. Agent according to claims 4, 5, 6, 7 and 8, **characterised in that** alpha-ketoglutaric acid is present in a concentration of 3-20 g/l, 5-hydroxymethylfurfural is present in a concentration of 1-3 g/l, glucose is present in a concentration of 20-100 g/l, sodium ions are present in a concentration of 60-160 mmol/l and potassium ions in a concentration of 15-40 mmol/l.

10. Agent according to claim 9, **characterised in that** alpha-ketoglutaric acid is present in a concentration of 6-16 g/l, 5-hydroxymethylfurfural is present in a concentration of 1-2.5 g/l, glucose is present in a concentration of 20-50 g/l, sodium ions are present in a concentration of 70-160 mmol/l and potassium ions are present in a concentration of 20-40 mmol/l.

11. Agent according to any one of claims 1 to 5 or 7, **characterised in that** it is present in solid or liquid administration form, to be administered orally or rectally, which contains the ketoglutaric acid at least partly in the form of its monosodium salts or monopotassium salts.

12. Agent according to claim 11, **characterised in that** it contains diluting agents and/or extenders and/or disaccharides, in particular icing sugar to improve the taste.

13. Agent according to claim 11 or 12, **characterised in that** the dosage units contain 3-9 g alpha-ketoglutaric acid, 0.5-1.5 g 5-hydroxymethylfurfural, 1.4-2.3 mg N-acetyl-seleno-L-methionine and 70-230 mg N-acetyl-L-methionine.

14. Method for producing an agent in a form suitable for intravenous administration according to any one of claims 8 to 10, **characterised in that** the alpha-ketoglutaric acid is dissolved at an elevated temperature in distilled water which has been made low in oxygen by gassing, the solution thus obtained is adjusted to a pH of slightly above 4 after the possible addition of glucose or fructose by the addition of alkalis, but not ammonia or amines, and the N-acetyl-seleno-L-methionine, the N-acetyl-L-methionine and the compound that is capable of forming azomethine are then added to this mixture.

15. Method for producing preparations suitable for oral or rectal administration according to any one of claims 11 to 13, **characterised in that**, to adjust a pH of 3-6, the ketoglutaric acid is used either partly or completely in the form of its monosalt with sodium and/or potassium and is mixed with extenders and, if desired, also with disaccharides, whereupon the substance that is capable of forming azomethine, the N-acetyl-seleno-L-methionine and the N-acetyl-L-methionine are added to the mixture, whereupon the mixture is converted into the desired administration form in particular into drinking granules, into tablets or an irrigation means.

16. Agent according to any one of claims 1 to 13 as a pharmaceutical agent.

17. Use of the substances cited in claims 1 to 11 for the production of a pharmaceutical agent for malignant tumours.

## Revendications

1. Composition ayant une action destructrice sur les tumeurs malignes, qui contient en tant qu'agent actif un acide alpha-cétoglutarique (XX) ou un sel pharmaceutiquement acceptable en dérivant et au moins un composé susceptible de donner formation à une liaison azométhinique (ZZ) par une réaction enzymatique dépendante, appartenant au groupe comprenant: le 5-hydroxyméthylfurfural, l'acide déhydroascorbinique, le maltol et la vanilline, dans laquelle de préférence le rapport massique XX/ZZ est supérieur à 1/1, notamment compris entre 2/1 et 12/1, **caractérisée en ce que** la composition contient à titre de constituant actif supplémentaire la N-acétyl-seleno-L-méthionine et la N-acétyl-L-méthionine, cette dernière étant employée en excès par rapport à la N-acétyl-L-séléno-L-méthionique

2. Composition selon la revendication 1, **caractérisée en ce que** le rapport massique XX/ZZ est compris entre 100/1 et 20 000/1, de préférence 500/1 à 10000/1.

3. Composition selon la revendication 1 ou 2 **caractérisée en ce que** le rapport massique XX/ZZ est compris entre 20/1 et 300/1, de préférence 500/1 à 100/1.

4. Composition selon une quelconque des revendications 1 à 3, **caractérisée en ce que** qu'elle contient en tant que composant supplémentaire du glucose, du fructose ou leurs mélanges.

5. Composition selon une quelconque des revendications 1 à 4, **caractérisée en ce que** ZZ est le 5-hydroxyméthylfurfural.

6. Composition selon une quelconque des revendications 1 à 5, **caractérisée en ce que** ladite composition est sous forme de solution aqueuse et **en ce que** la N-acétyl-séléno-L-méthionine est employée en une proportion comprise entre 1,4 à 2,3 mg/litre et la N-acétyl-L-méthionine dans une proportion comprise entre 70 et 230 mg/litre.

7. Composition selon une quelconque des revendications 1 à 6, **caractérisée en ce qu'**elle contient un électrolyte consistant en sodium ou potassium.

8. Composition selon une quelconque des revendications 1 à 7 **caractérisée en ce qu'**elle est administrable par voie intraveineuse et **en ce que** son pH est compris entre 4 et 6.

9. Composition selon une quelconque des revendications 4, 5, 6, 7 et 8, **caractérisée en ce qu'**elle contient de 3 à 20 g/litre d'acide alphacétoglutarique, de 1 à 3 g/litre de 5-hydroxyméthylfurfural, de 20 à 100 g/litre de glucose, de 60 à 160 mmoles d'ions sodium et de 15 à 40 mmoles d'ions potassium.

10. Composition selon la revendication 9, **caractérisée en ce qu'**elle contient de 6 à 16 g/litre d'acide alphacétoglutarique, de 1 à 2,5 g/litre de 5-hydroxyméthylfurfural, de 20 à 50 g/litre de glucose, de 70 à 160 mmoles d'ions sodium et de 20 à 40 mmoles d'ions potassium.

11. Composition selon une quelconque des revendications 1 à 5 ou 7, **caractérisée en ce qu'**elle est sous une forme solide ou liquide, susceptible d'être administrée par voie orale ou rectale, dans laquelle l'acide XX est au moins en partie sous la forme d'un sel monosodique ou monopotassique.

12. Composition selon la revendication 11, **caractérisée en ce qu'**elle contient des agents de dilution et/ou d'extension et/ou d'amélioration du goût de type disaccharide, notamment du sucre pulvérulent.

13. Composition selon la revendication 11 ou 12, **caractérisée en ce que** les doses unitaires contiennent 3 à 9 g de XX, 0,5 à 1,5 g de 5-hydroxyméthylfurfural, 1,4 à 2,3 mg de N-acétyl-séléno-L-méthionine et 70 à 230 g de N-acétyl-L-méthionine.

14. Procédé de fabrication d'une composition destinée à être administrée sous une forme convenant à une administration intraveineuse selon une quelconque des revendications 8 à 10, **caractérisée en ce que** l'XX est dissous à haute température dans de l'eau distillée, dans de l'eau rendue appauvrie en oxygène par aération, laquelle solution obtenue, après addition de glucose ou de fructose, est portée à un pH d'environ 4 par addition d'un produit alcalin ne consistant cependant ni en ammoniaque, ni en amine, et **en ce que** le mélange ainsi obtenu est alors additionné de N-acétyl-séléno-L-méthionine, de N-acétyl-L-méthionine et de ZZ.

15. Procédé de fabrication d'une préparation destinée à être administrée par voie orale ou rectale selon une quelconque des revendications 11 à 13, **caractérisé en ce que**, pour l'obtention d'un pH de l'ordre de 3 à 6, est ajoutée de la XX en totalité ou en partie, sous forme monosodique et/ou monopotassique, puis on ajoute par mélange un agent d'expansion ou, selon le cas, également des disaccharides, après quoi on ajoute au mélange de la ZZ, de la N-acétyl-séléno-L-méthionine et de la N-acétyl-L-méthionine, après quoi ce mélange est mis sous la forme convenant à son administration, notamment sous la forme de granulats solubles, de comprimés ou sous une forme à pulvériser.

16. Composition selon une quelconque des revendications 1 à 3, agissant en tant que moyen médicamenteux.

17. Application du composé selon une quelconque des revendications 1 à 11, pour l'obtention d'un médicament susceptible d'agir à l'encontre des tumeurs malignes.
